# EUROPEAN PATENT SPECIFICATION

(11) **EP 3 182 913 B1**
(45) Date of publication and mention of the grant of the patent: **16.10.2019**
(21) Application number: 15767276.7
(22) Date of filing: 30.07.2015
(51) Int. Cl.: A61B 17/56, A61M 39/10

(54) **DEVICE FOR SUPPLYING FLUID SUBSTANCES IN THE BODY OF A PATIENT**
VORRICHTUNG ZUR BEREITSTELLUNG VON FLUIDSUBSTANZEN IM KÖRPER EINES PATIENTEN
DISPOSITIF POUR ALIMENTER EN SUBSTANCES FLUIDES LE CORPS D'UN PATIENT

(30) Priority: 20.08.2014 IT VR20140214
(43) Date of publication of application: 28.06.2017
(73) Proprietor: Tecres S.P.A., 37066 Sommacampagna (VR) (IT)
(72) Inventor: FACCIOLI, Giovanni, I-37066 Sommacampagna (Verona) (IT); SOFFIATTI, Renzo, I-37066 Sommacampagna (Verona) (IT)
(74) Representative: Feltrinelli, Secondo Andrea
(86) International application number: PCT/IB2015/055786
(87) International publication number: WO 2016/027190

(56) References cited:
- WO-A2-2006/023430
- US-A- 5 591 171
- US-A1- 2004 122 438
- US-A1- 2013 109 999

## Description

### TECHNICAL FIELD OF THE INVENTION

The present invention regards a device for supplying fluid substances in the body of a patient, for example for supplying or feeding a bone cement into a bone cavity, such as a patient's vertebra.

### STATE OF THE PRIOR ART

Up to now, many devices have been proposed for supplying bone cement in the vertebrae of patients.

The international application WO2004057280A1, on behalf of the applicant of the present patent application, teaches a surgical device for injecting bone cement in a bone cavity of a patient; such device comprises a hollow elongated element to be partially inserted in the bone cavity, a connector component in communication with a bone cement source and rotatably mounted on the hollow elongated element.

The United States patent application US20040260303A1 instead teaches a device comprising a first element connected to a bone cement syringe or container and a second element pivoted to the first and connected to a needle to be inserted in a bone cavity of a patient.

Both above-indicated solutions, if on one hand ensure a certain relative mobility of the respective components, even during the injection of the bone cement, in any case have several structural constraints, which partly limit the freedom of action of the doctor or operator, as well as the adaptability to different operation conditions. Moreover, with such devices, the doctor or the operator who is executing the injection cannot - unless another doctor or operator is assisting - move away from the operation area, without interrupting the injection.

WO2006023430A2 and US20040122438 teach connectors according to the state of the technique.

### OBJECTS OF THE INVENTION

One object of the present invention is to provide a new device for supplying fluid substances in the body of a patient.

Another object of the present invention is to provide a device for supplying fluid substances in the body of a patient that ensures good adaptability and versatility.

Another object of the present invention is to provide a device for supplying fluid substances in the body of a patient due to which the doctor can also move away from the operation area, without interrupting the injection.

In accordance with the invention, devices according to claims 1 and 10 are provided.

The dependent claims refer to preferred and advantageous embodiments of the invention.

### BRIEF DESCRIPTION OF THE DRAWINGS

Other characteristics and advantages of the invention will be clearly seen in the description of embodiments of a device, illustrated by way of example in the enclosed drawings in which:
- figure 1 is a slightly top perspective view of a device according to the present invention;
- figure 2 is an exploded view of the device of fig. 1;
- figure 3 is a slightly top perspective view of the device of fig. 1 provided with a first component of connector or connection to a syringe;
- figure 4 is a slightly top perspective view of a unit for supplying a fluid substance according to the present invention provided with the device of fig. 1;

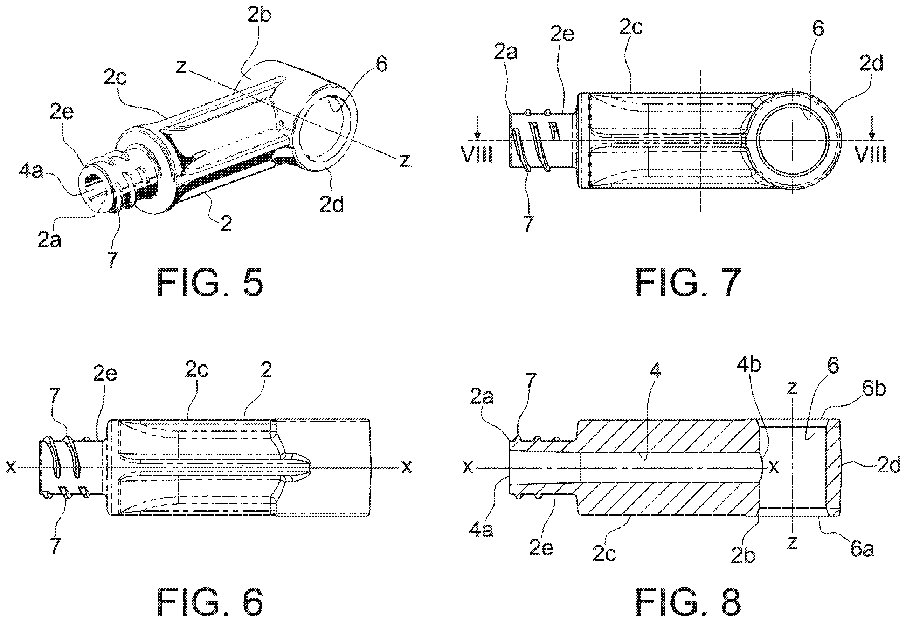

- figure 5 is a slightly top perspective view of a first tubular element of a device according to the present invention;
- figures 6 and 7 are side views with parts in section of the first tubular element of fig. 5;
- figure 8 is a sectional view along the line VIII-VIII of fig. 7;

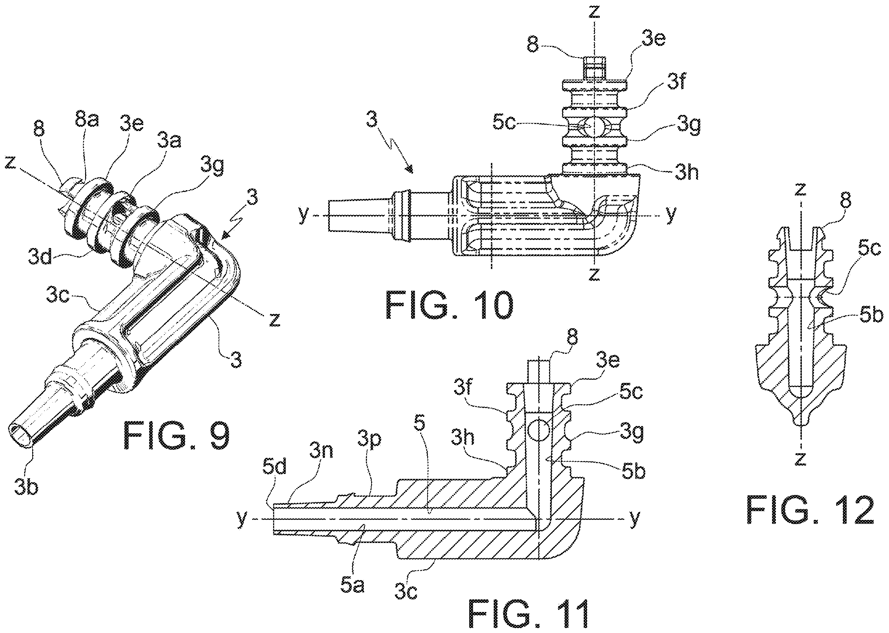

- figure 9 is a slightly top perspective view of a second tubular element of a device according to the present invention;
- figure 10 is a side view of the second tubular element of fig. 9;
- figures 11 and 12 are sectional views of the second tubular element of fig. 9;

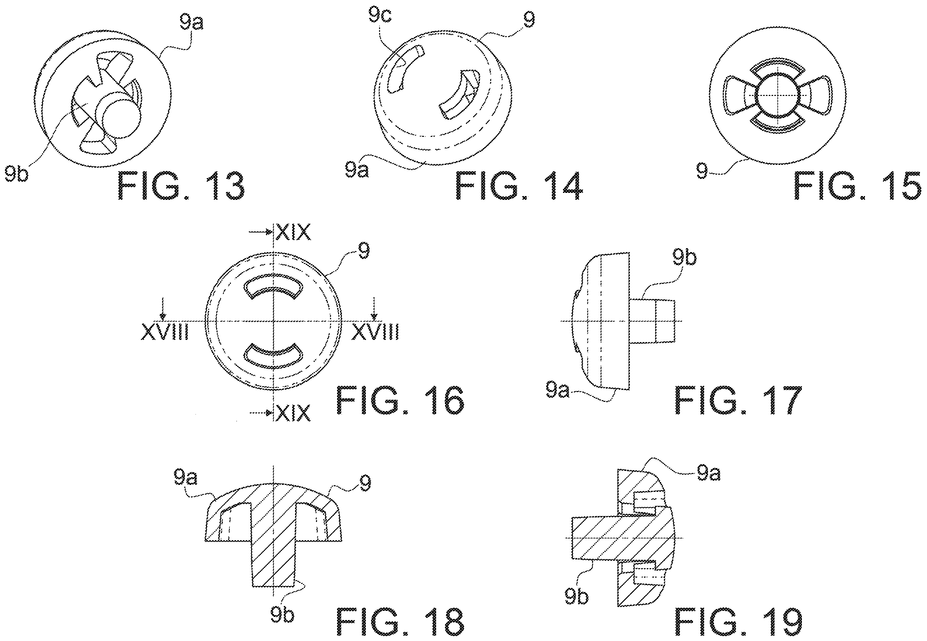

- figures 13 and 14 are slightly top perspective views, from respective sides, of a cap component of a device according to the present invention;
- figures 15, 16 and 17 are respectively top, bottom and side views of the cap component of figure 13;
- figures 18 and 19 are sectional views along the lines, respectively XVIII-XVIII and XIX-XIX of the cap component of figure 13;
- figure 20 is a slightly top perspective view of another device according to the present invention;
- figure 21 is an exploded view of the device of fig. 20;
- figures 22 and 23 are respectively lateral and front views of the device of figure 20;
- figures 24 and 25 are sectional views respectively along the lines XXIV-XXIV and XXV-XXV of the device of figure 22;
- figure 26 is a slightly top perspective view of a unit for supplying a fluid substance according to the present invention provided with the device of fig. 20;
- figure 27 is a slightly top and exploded perspective view of another device according to the present invention;
- figures 28 and 29 illustrate respective steps of use of a device according to the present invention.

In the set of drawings, equivalent parts or components are marked with the same reference numbers.

### EMBODIMENTS OF THE INVENTION

With reference to figures from 1 to 19, a device or joint 1 is illustrated for supplying fluid substances in the body of a patient according to the present invention comprising a first tubular element 2 and a second tubular element 3 that can be angularly moved with respect to each other, as will be better clarified hereinbelow.

The first tubular element 2 has an end of connection 2a to a source of a fluid substance, such as bone cement BC, and a supply end 2b and such element 2 then defines a first flow channel 4 for conveying a fluid substance from the end of connection 2a to the supply end 2b. In addition, the first tubular element 2 has at least one first segment 2c that extends along or around a first longitudinal axis x-x, if desired a longitudinal symmetry axis of the first segment 2c, such first segment 2c delimits at least part of the first flow channel 4.

The second tubular element 3 instead has an inlet end 3a for the fluid substance as well as an outlet end 3b for feeding the fluid substance outside the device 1 and if desired within or towards a needle to be inserted in the body of a patient, for example in a bone cavity thereof, as will be better described hereinbelow. The second tubular element 3 defines a second flow channel 5 in fluid communication with the first flow channel 4 and intended to convey a fluid substance from the inlet end 3a to the outlet end 3b. The second tubular element 3 also has at least one first section 3c extended along or around a second longitudinal axis y-y, if desired a longitudinal symmetry axis of the first section 3c, such first section 3c at least partly delimiting the second flow channel 5.

The first tubular element 2, moreover, is angularly movable with respect to the second tubular element 3 around an articulation axis z-z transverse or orthogonal to the first segment 2c of the first tubular element 2 and, if desired, to the first section 3c. According to the embodiment illustrated in the figures, the articulation axis z-z is substantially orthogonal both to the first longitudinal axis x-x and to the second longitudinal axis y-y.

According to the invention, one or more articulation or angular movement components 2d, 3d are provided for the angular movement or articulation of the first tubular element 2 with respect to the second tubular element 3 around an articulation axis z-z, such articulation or movement component/s 2d, 3d extending or developing transverse or orthogonal to the first segment 2c of the first tubular element 2 and extends/extend along the articulation axis z-z so as to offset the first segment 2c of the first tubular element 2 and the first section 3c of the second tubular element 3 in the direction of the articulation axis z-z. If desired, the articulation or movement component/components 2d, 3d is/are also transverse or orthogonal to the first section 3c of the second tubular element 3.

This offset is such to ensure the non-alignment of the first segment 2c of the first tubular element 2 with respect to the first section 3c of the first tubular section 3, so as to ensure the rotation or angular movement of the first tubular element 2 around the articulation axis z-z without the second tubular element 3 interfering with or obstructing such movement.

If desired, the first segment 2c of the first tubular element 2 is articulated with respect to the second tubular element 3 in a manner such to define an articulation plane AP orthogonal to the articulation axis z-z and passing through the first longitudinal axis x-x. In such case, the first section 3c of the second tubular element 3 is offset with respect to the articulation plane PA, more particularly it can be offset by a distance at least or, preferably, substantially equal to the transverse size of the first segment 2c of the first tubular element 2.

In substance, due to the articulation or angular movement components 2d, 3d, the first tubular element 2 is rotatably and projectingly supported, along the direction of the articulation axis z-z, by the second tubular element 3.

Advantageously, the inlet end 3a of the second tubular element 3 is articulated to the supply end 2b of the first tubular element 2 around the articulation axis z-z.

As will be understood, this can be obtained by directly pivoting or articulating, i.e. without intermediate components, the inlet end 3a to the supply end 2b, or by providing or arranging an intermediate component on one side constrained to one of the tubular elements 2 or 3 and on the other side rotatably anchored or articulated to the other of the tubular elements 3 or 2.

According to the invention, angular movement or articulation component/s comprises/comprise a shank component 3d of one from among the first tubular element 2 and the second tubular element 3 and defining the inlet end 3a or the supply end 2b, as well as an annular or cradle component 2d of the other from among the second tubular element 3 and the first tubular element 2 and defining the other from among the supply end 2b or the inlet end 3a. The annular or cradle component 2d then delimits a seat 6 for the housing and articulated connection of the shank component 3d around the articulation axis z-z, so as to allow the articulation of the supply end 2b with respect to the inlet end 3a around the articulation axis z-z.

If desired, the shank component 3d is extended transversely or orthogonally from the first segment 2c of the first tubular element 2 or from the first section 3c of the second tubular element 3, so as to respectively define a second segment of the first tubular element 2 or a second section of the second tubular element 3.

According to the invention, the first tubular element 2 or the second tubular element 3 is substantially configured as an elbow, with one arm of the elbow comprising the first segment 2c or the first section 3c, while the other arm of the elbow includes the shank component 3d.

Preferably the annular or cradle component 2d like the shank component 3d is extended along or around the articulation axis z-z, which can constitute a longitudinal axis, if desired a longitudinal symmetry axis of the annular or cradle component 2d and/or of the shank component 3d.

Preferably, the first tubular element 2 is formed in a single piece, if desired via molding. For such purpose and with reference to the non-limiting embodiment illustrated in the figures, from the end of connection 2a to the supply end 2b, the first tubular element 2 comprises the first segment 2c and then the annular or cradle component 2d, which for example can include a substantially cylindrical segment extended transversely or orthogonally starting from the terminal end of the first segment 2c and internally defines the seat 6 for housing and articulated connection. The substantially cylindrical segment 2d and the housing and articulated connection seat 6 have longitudinal symmetry axis, preferably orthogonal to the first longitudinal axis x-x, which during use coincides with the articulation axis z-z. If desired, the first tubular element 2 comprises a sleeve component 2e defining the end of connection 2a, then the first segment 2c, for example with transverse external diameter or section greater than the sleeve component 2e and then the annular or cradle component 2d.

With one such structure, the first flow channel 4 is at least partly delimited by the first segment 2c and, if provided, partly by the sleeve component 2e and then has an inflow opening 4a at the end of connection 2a, delimited by the first segment 2c or, if provided, by the sleeve component 2e, as well as a terminal opening 4b, at the supply end 2b, delimited by the first segment 2c. At the terminal opening 4b, the first flow channel 4 opens into the housing and articulated connection seat 6, for conveying fluid, as will be better described hereinbelow, into the second flow channel 5. If desired, the first flow channel 4 has substantially constant diameter or section for the entire extension thereof and can be substantially straight or rectilinear.

The housing and articulated connection seat 6 instead preferably has cylindrical configuration with substantially constant section, if desired open at both ends 6a, 6b thereof.

The first tubular element 2, or if desired and if provided the sleeve component 2e, can have means 7 for anchoring, for example an externally threaded portion or a bayonet anchoring portion, to a syringe 11 or to a first connector or connection component 10a, in turn connected to a syringe 11.

Advantageously, the second tubular element 3 is formed in a single piece, if desired via molding. For such purpose and with reference to the non-limiting embodiment illustrated in the figures, the second tubular element 3 comprises, from the inlet end 3a to the outlet end 3b, the first section 3c and the shank component 3d, the latter preferably extended transversely or orthogonally from the first section 3c and both delimiting a respective first 5a and second 5b branch, transverse or orthogonal to each other, of the second flow channel 5, preferably having diameter or section that is constant or substantially equal to each other. With regard in particular to the shank component 3d, this has an internal wall delimiting the second branch 5b of the second flow channel 5 and an external wall suitably shaped so as to be insertable within the housing and articulated connection seat 6. Starting from the external wall of the shank component 3d or from the internal surface of the cradle or annular component 2d, two or more annular ribs 3e, 3f, 3g and 3h can be extended, e.g. three or four, which are suitably spaced, for a reason that will be described hereinbelow, and have external diameter and section such to be insertable to size within the housing and articulated connection seat 6.

In the wall of the shank component 3d, one or more through holes 5c are also formed that are intended to place in fluid communication the zone outside the shank component 3d with the second flow channel 5, more in detail with the second branch 5b of the second flow channel 5. More particularly, during use, the through hole/s 5c is/are intended to place in fluid communication the first flow channel 4 and the second flow channel 5. The through hole/s 5c can be obtained in one side of the shank component 3d delimited between two respective annular ribs 3f and 3g.

If desired, the second tubular element 3 comprises, from the inlet end 3a to the outlet end 3b, a bush component 3n, 3p defining the outlet end 3b, then the first section 3c, e.g. with external transverse section or diameter greater than the bush component 3n, 3p and then the shank component 3d. The bush component 3n, 3p can comprise a first externally tapered wall 3n and then a externally substantially cylindrical part 3p close to the first section 3c.

With one such structure, the second flow channel 5 is at least partly delimited by the first section 3c and, if provided, partly by the bush component 3n, 3p and has an outlet opening 5d, delimited by the first segment 3c or, if provided, by the bush component 3n, 3p, as well as an inlet opening 5c delimited by the shank component 5d and for example corresponding to the aforesaid hole/s 5c.

At the first section 3c or, if provided, at the bush component 3n, 3p, a needle 12 can then be connected, directly or by means of interposition of a second connector or connection component or nut 10b, such needle 12 to be inserted in the body of a patient, as will be described hereinbelow, for example a needle 12 with bevel tip 12, which has an opening substantially lateral with respect to the longitudinal extension of the needle. To this regards, a device according to the present invention comprises means for removably fixing the outlet end 3b to a needle 12 or, if provided, a secondo connector component or nut 10b, the outlet end 3b to the second connector component 10b and the latter to a needle 12, so that it is possible to removably fix the device 1, or better the secondo tubular component 3 to the needle 12 itself. For such purpose, the means for removably fixing can include, for example, a thread or snap-fit engagement or another type of engagement, but in such a way as after having constrained the device to the needle, the second tubular component 3 and the needle 12 cannot rotate with respect to each other or mutually, unless the same components are deliberately disconnected (with interposition or with no interposition of a second connector component 10b), particularly at the end of the bone cement BC supply. Then, in such case, the needle 12 could rotate, together with the second tubular component 3, with respect to the first tubular component 2 about the articulation axis z-z only, but not about the second longitudinal axis y-y, and the rotation of the needle 12 about this axis y-y could occur only by rotating the whole unit 16, which will be better described in the following.

The second tubular component 3 can then comprise suitable engagement means or components 8, 9 due to which it is possible to connect, with rotatable engagement, the shank component 3d to the annular or cradle component 2d, such that the latter can be connected or removably connected to the shank component 3d, but angularly movable with respect thereto around the articulation axis z-z. The engagement means or components can comprise one or a pair of tabs or protuberances 8, if desired elastically yielding with free end curved so as to delimit a tooth or a shoulder 8a; the tabs or protuberances 8 are for example extended from the end of the shank component 3d far from the first section 3c, which actually corresponds to the inlet end 3a of the second tubular element 3.

The means for the engagement and rotatable connection of the shank component 3d to the annular or cradle component 2d comprise a cap component 9 intended to engage, if desired removably, the tabs or protuberances 8 of the shank component 3d, such to rotatably anchor the shank component 3d to the annular or cradle component 2d around the articulation axis z-z. For such purpose, the cap component 9 can have a base segment 9a as well as a stem segment 9b insertable within the seat 6, but on an opposite side with respect to the end 6a of insertion of the shank component 3d, i.e. within the end 6b so as to engage the latter and maintain it inserted in position within the housing and articulated connection seat 6.

More particularly, in the base segment 9a of the cap component 9, one or more openings 9c is formed for the insertion and locking, if desired snap insertion/locking, of a respective tab or protuberance 8.

Of course, the engagement means could also be different from those described above and illustrated in the figures, e.g. one or more annular grooves formed in the housing and articulated connection seat 6 as well as one or more elastically yielding tabs extended from the shank component and intended to slidable engage one or a respective annular groove. Alternatively, the shank component 3d could be substantially inserted to size within the housing and articulated connection seat 6, but with the possibility of mutually rotating such components by means of application of a suitable force.

A device according to the present invention, therefore, in accordance with the specific non-limiting embodiment illustrated in the figures, provides for a first tubular element 2 and a second tubular element 3 connected, for example by means of insertion of the shank component 3d of the second tubular element within the housing and articulation seat 6, and then anchored in position within the same by means of engagement means 8, 9.

In addition, the device can comprise suitable sealing means for the fluid seal connection between the first flow channel and second flow channel. For such purpose, between the annular or cradle component 2d and the shank component 3d, one or more gaskets 13a, 13b, if desired O-ring, are preferably housed.

The sealing means can, for example, comprise at least two annular gaskets 13a, 13b mounted on the shank component 3d so as to delimit with the cradle or annular component 2d and with the shank component 3d a first zone of the housing and articulated connection seat 6. In this case, the first flow channel 4 opens in the first zone of the housing and articulated connection seat 6 and the at least one through hole 5c opens in the first zone.

If desired, the sealing means comprise a first gasket 13a between two respective annular ribs 3e and 3f and a second gasket 13b between two other respective annular ribs 3g and 3h. In such case, the first flow channel 4 opens into a zone of the housing and articulated connection seat 6 between the two annular ribs 3f and 3g delimiting the part of the shank component 5d in which the hole/s 5c is/are obtained, such that a fluid substance fed into the flow channel 4, crosses the latter, exits from this at the terminal opening 4b, entering into the annular band of the housing and articulated connection seat 6 delimited between the annular or cradle component 2d and the shank component 3d and is introduced into the second flow channel 5 through the hole/s 5c.

As stated above, one such device can be connected on one side, at the end of connection 2a, to a syringe 11 or to a first connector or connection component 10a, in turn connected to a syringe 11, so as to place the first flow channel 4 in fluid communication with the internal volume of the syringe 11, and on the other side, at the outlet end 3b, to a needle 12, if desired to a second connector or connection component 10b in turn connected to a needle 12, so as to place the second flow channel 5 in fluid communication with the internal gap of the needle 12, to be introduced and inserted in the body of a patient, for example in a vertebra thereof for the insertion of bone cement in such patient. The fluid substance can then be thrust from the syringe 11, into the device 1 and then into the needle 12 and into the patient, by operating on a suitable knob 13 intended to actuate a thrust means for the fluid substance into the syringe 11, such as a screw or the like 14 acting on a piston (not visible in the figures) or another similar component slidably mounted in the syringe 11. Additionally, the operator can maintain the syringe 11 at a grip 15 in which the screw 14 or the syringe 11 itself is fit.

A device according to the present invention assembled as stated above with a syringe 11 and a needle 12, constitutes a unit 16 for supplying a fluid substance according to the present invention.

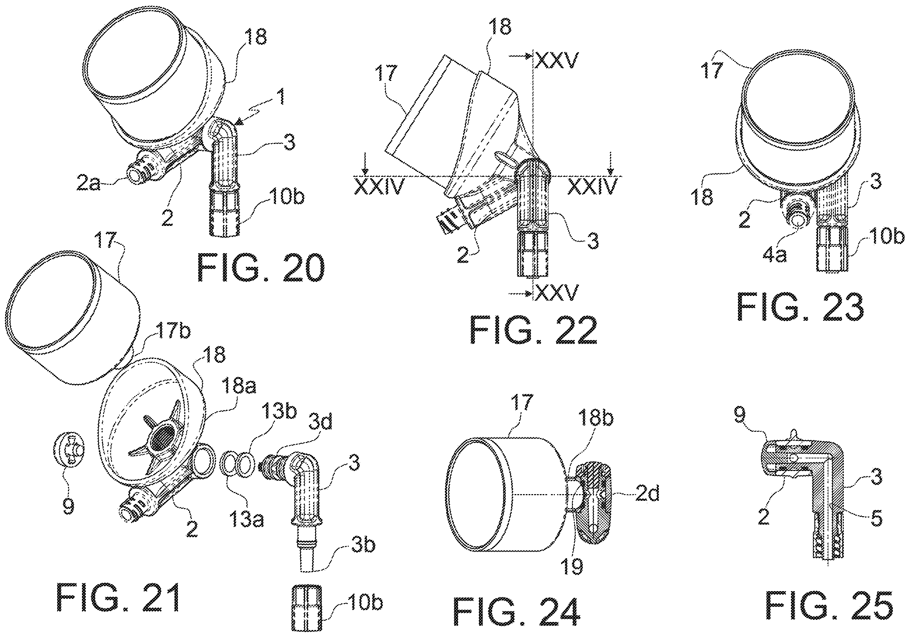

According to a variant illustrated in figures 20 to 26, a device according to the present invention also provides for a pressure measurement device, such as a manometer 17 intended to measure the pressure of the fluid substance in the flow channel 4 or 5. For such purpose, the device can include a support component 18 for the manometer 17, mounted on the device, if desired connected or formed in a single piece with the first tubular element 2, if desired extended from the annular or cradle component 2d of the same. The support component 18 can have a cap 18a that during use houses part of the body 17a of the manometer 17 as well as a protuberance 18b delimiting a hole, if desired threaded, for engaging via screwing a shank 17b, if desired externally threaded, of the manometer 17. A membrane 19 is then provided, housed between the support component 18 and the first 4 or second 5 flow channel, which is suitably stressed by the fluid in the flow channel 4 or 5 and determines a corresponding pressure measurement by the manometer 17.

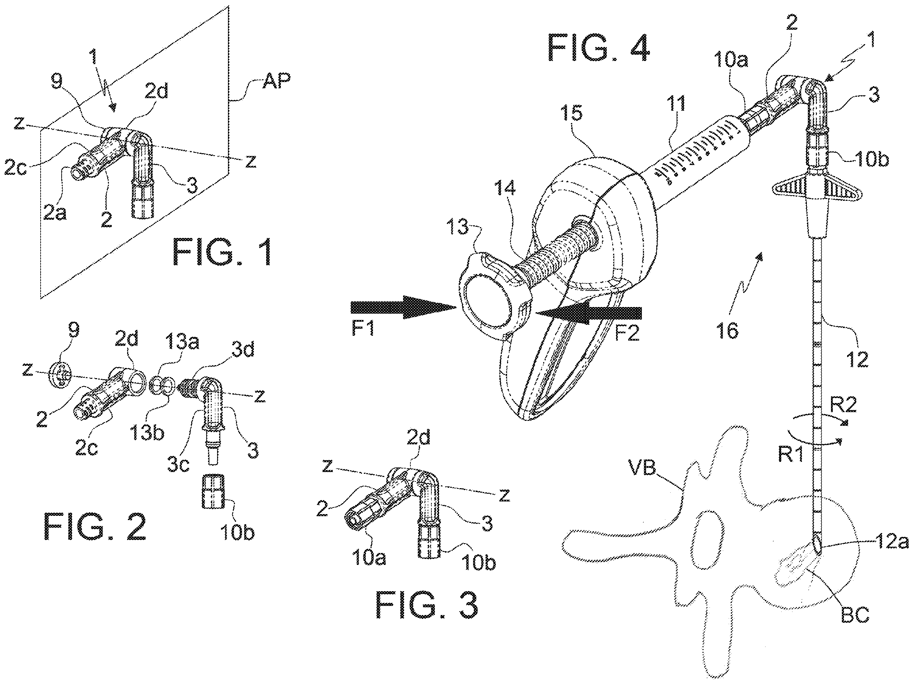

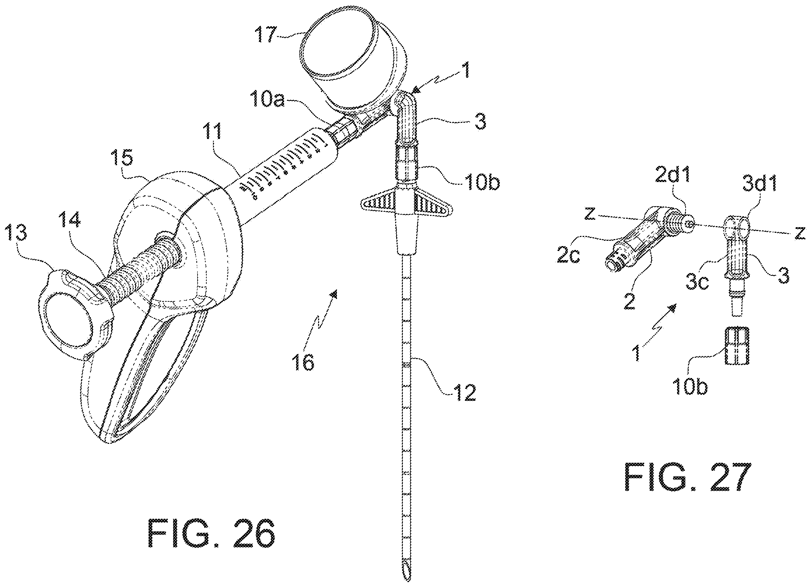

With reference now to figure 27, a device according to the present invention is shown that is similar to the device of fig. 1, but in which the shank component 2d1 is part of the first tubular element 2 and is extended from the first segment 2c of the latter, while the annular or cradle component 3d1 is part of the second tubular element 3 and is extended from the first section 3c of the same.

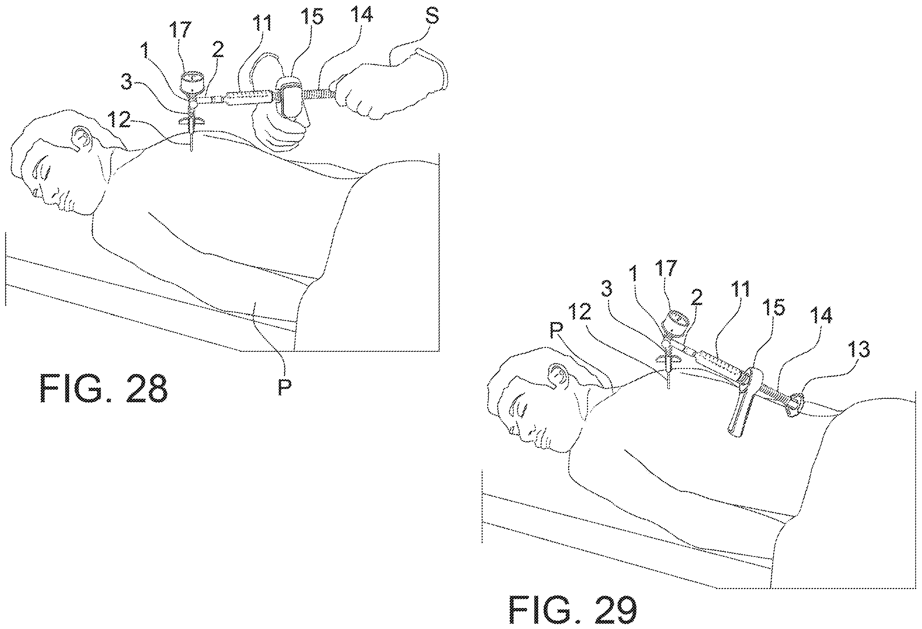

With a device according to the present invention, suitably connected with a syringe 11 and a needle 12, one inserts or introduces the needle 12 inside the body of the patient P, for example in a vertebra thereof and hence the doctor or operator S operates on the syringe 11 so as to insert the fluid substance within the body of the patient (see figure 28). During such step, the doctor or operator S holds the syringe 11 lifted.

If the doctor or operator S should move away from the patient P, at least temporarily leaving the device 1, so as to rest it, for example on the back of the patient P (see figure 29), or move the device 1, for example to bring the syringe 11 higher or lower or on the opposite side of the patient P, this could be easily attained due to the expedient according to which the first section 3c of the second tubular element 3 is offset as stated above. Such characteristic of a device according to the present invention in fact allows obtaining an articulation or angular movement of the first tubular element 2 with respect to the second tubular element 3, without any interference or obstruction by the second tubular element. Such articulation also allows obtaining an angular movement between the two tubular elements 2 and 3 for a wide range or angular interval, e.g. 180°, if desired also 270° or even 360° around the articulation axis z-z.

In addition, with a device according to the present invention, good visibility of the operation zone can also be obtained, considering the fact that the two tubular elements 2, 3 are not actually aligned in the same articulation plane.

It should also be noted that a device according to the present invention is easy to achieve as well as assemble, considering the fact that in order to connect the two tubular elements, it is sufficient, according to the non-limiting embodiment illustrated in the drawings, to insert the shank component 3d in the housing and articulated connection seat 6, if desired with interposition of gaskets, and then engage the engaging means.

With the devices proposed up to now, instead, it is not possible to obtain a satisfactory articulation between respective components. In particular, with regard to US20040260303A1, it teaches a device with a first element and a second element pivoted to the first, but it is not clear how the same are pivoted, and in any case from what can be understood, the described structure provides for limits of articulation and pivoting between the components as well as a complex assembly thereof.

With reference instead to WO2006023430A2, first of all, such document teaches a device for supplying comprising a rotatable adaptor or connector including a first and a second tubular section, as well as a first and a second hollow housing and in which the first tubular section is mounted for rotation about a first axis with respect to the first hollow housing, whereas the second tubular section is mounted for rotation about a second axis with respect to the second hollow housing, which second axis is orthogonal to the first axis.

As it will be appreciated, such device for supplying comprises a structure much more complex and having more components than a device according to the present invention, and this clearly implies advantages both for manufacturing and for assembling the device in accordance with the present invention with respect to the solution of the prior art document now being examined.

Moreover, in the device of WO2006023430A2 the components rotate about two articulation axes and, more particularly, an articulation even with respect to the needle occurs, which, when the bone cement is inserted, could render the localization of the trim of the needle bevel tip by the operator or the surgeon very complex. On the contrary, a device according to the present invention, in accordance with a preferred variant, comprises means for removably fixing to a needle, and, owing to such an expedient, it is always possible to know, during the treatment, the arrangement of the needle and of the respective bevel tip, thereby obtaining clear advantages both of safety and treatment speed kind.

With reference to such an aspect, the needle which is preferred during the vertebroplasty or kyphoplasty is the so-called with bevel tip or with tip 12a shaped as a "flute spout", which tip 12a makes it possible to enter with high precision into the soft tissues and the vertebral bone. This tip, owing to its substantially flat shape, acts as a guide and when it is suitable moved, it allows the doctor to reach the point in the vertebral body pre-established for inserting a substance, such as bone cement BC. Moreover, taking into consideration that the bevel tip has an opening actually lateral, it makes it possible to laterally supply cement.

If one moves or better determines an angular movement of the needle 12 and thus of the tip 12a, it is possible to supply cement to different points of a vertebral body VB. It may happen that the cement is supplied at a slit of the vertebral body VB and this event, which is not rare, represents a serious danger for the patient, since the cement, in this case, could come out the vertebral body VB, thereby causing very serious, and even lethal, damages. In such case, if a needle with bevel tip is provided, it is sufficient to rotate the needle, even by few degrees about its longitudinal axis, so as to move the cement away from the slit in the vertebral body VB, thereby actually eliminating the danger.

It is derivable from the above that the doctor should maintain a complete control of the position of the needle even referring to the position of the lateral opening of the bevel tip 12a. According to a preferred variant of the invention subject-matter of the present patent application, the device for supplying is firmly fixed or fixable to a needle 12 and therefore the doctor always knows where the tip 12a of the latter is faced, irrespectively from the manoeuvre being made and it is not possible for the tip 12a to be accidentally moved and moved independently from the device for supplying.

To this regard, in fact, should a force F1 be applied to the know 13, the unit 16 and thus, in turn, the needle 12 would be rotated about the axis y-y or about an axis parallel thereto in a direction of rotation R1, whereas should a force F2 be applied to the knob 12 in a direction opposite to to the force F1, the unit 16 and thus, in turn, the needle 12 would always be rotated about the axis y-y or about an axis parallel thereto but with a direction of rotation R2 opposite to R1.

On the contrary, in accordance with the solutions of the state of the technique and particularly of WO2006023430A2, the needle is free to rotate relatively to the respective device for supplying and this since no constrain or fixing between such components is provided. This fact, as above indicated, is very dangerous, since the tip is free to randomly rotate and the doctor cannot exactly establish where the cement will come out and the doctor would need of the help of a second operator arranged to keep the needle in position. Moreover, even in this case, the supply of cement will be inaccurate and not satisfactory, since it will not be established by the doctor, but by another person or with the help of another person.

Modifications and variants of the invention are possible within the protective scope defined by the claims.

## Claims

1. Device for supplying fluid substances in the body of a patient, comprising:
- a first tubular element (2) having an end of connection (2a) to a source of a fluid substance and a supply end (2b), said first tubular element (2) defining a first flow channel (4) for conveying a fluid substance from said end of connection (2a) to said supply end (2b), said first tubular element (2) having at least one first segment (2c) extending along a first longitudinal axis (x-x);
- a second tubular element (3) having an inlet end (3a) as well as an outlet end (3b) for said fluid substance, said second tubular element (3) defining a second flow channel (5) in fluid communication with said first flow channel (4) and intended to convey a fluid substance from said inlet end (3a) to said outlet end (3b), said second tubular element (3) having at least one first section (3c) extending along a second longitudinal axis (y-y);
- at least one articulation or angular movement component (2d, 3d, 2d1, 3d1) for articulating or angularly moving said first tubular element (2) with respect to said second tubular element (3) around an articulation axis (z-z), said articulation axis being transverse or orthogonal to said first segment (2c) of said first tubular element (2), said at least one articulation or angular movement component (2d, 3d, 2d1, 3d1) extending along said articulation axis (z-z) so as to offset said first segment (2c) of said first tubular element (2) and said first section (3c) of said second tubular element (3) in the direction of said articulation axis (z-z), said at least one articulation or angular movement component (2d, 3d, 2d1, 3d1) comprising:
- a shank component (3d, 2d1) of one from among said first tubular element (2) and said second tubular element (3), said shank component (3d, 2d1) defining said inlet end (3a) or said supply end (2b), as well as
- a cradle or annular component (2d, 3d1) of the other from among said second tubular element (3) and said first tubular element (2), said cradle or annular component (2d, 3d1) defining the other from among said supply end (2b) or said inlet end (3a),
said cradle or annular component (2d, 3d1) delimiting a seat (6) for housing and articulated connection of said shank component (3d, 2d1) around said articulation axis (z-z), thereby allowing the articulation of said supply end (2b) with respect to said inlet end (3a) around said articulation axis (z-z),
wherein first tubular element (2) or said second tubular element (3) is substantially configured as an elbow, one arm of said elbow comprising said first section (3c) or said first segment (2c), whereas the other arm of said elbow includes said shank component (3d, 2d1),
wherein said device comprises engagement means (8, 9) for connecting, with rotatable engagement, said shank component (3d, 2d1) with said cradle or annular component (2d, 3d1),
wherein said second tubular element (3) comprises, from said outlet end (3b) to said inlet end (3a), said first section (3c) and said shank component (3d), said shank component (3d) extending transversely or orthogonally from the first section (3c) and both delimiting a respective first (5a) and second (5b) branch, transverse or orthogonal to each other, of said second flow channel (5), and
wherein said shank component (3d) has an internal wall delimiting said second branch (5b) of said second flow channel (5) and an external wall shaped so as to be insertable within said seat for housing and articulated connection (6), characterized that in the wall of said shank component (3d) at least one through hole (5c) is formed intended to place in fluid communication the zone outside said shank component (3d) and said second flow channel (5).

2. Device according to claim 1, **characterized in that** said at least one articulation or angular movement component (2d, 3d, 2d1, 3d1) is transverse or orthogonal to said at least one first segment (2c) of said first tubular element (2) and to said first section (3c) of said second tubular element (3).

3. Device according to claim 1 or 2, **characterized in that** said first segment (2c) of said first tubular element (2) is articulated with respect to said second tubular element (3) in a manner so as to define an articulation plane (PA) orthogonal to said articulation axis (z-z) and passing through said first longitudinal axis (x-x), and **in that** said first section (3c) of said second tubular element (3) is offset with respect to said articulation plane (PA).

4. Device according to any one of the preceding claims, **characterized in that** said inlet end (3a) of said second tubular element (3) is articulated to said supply end (2b) of said first tubular element (2) around said articulation axis (z-z).

5. Device according to any one of the preceding claims, **characterized in that** it comprises means for removably fixing said outlet end (3b) to a needle (12).

6. Device according to any one of the preceding claims, **characterized in that** from said end of connection (2a) to said supply end (2b), said first tubular element (2) comprises said first segment (2c) and then said cradle or annular component (2d), said cradle or annular component (2d) including a substantially cylindrical segment extended transversely or orthogonally starting from the terminal end of said first segment (2c) and internally defining said seat for housing and articulated connection (6).

7. Device according to claim 6, **characterized in that** said substantially cylindrical segment and said seat (6) for housing and articulated connection have longitudinal symmetry axis coinciding with said articulation axis (z-z).

8. Device according to any one of the preceding claims, **characterized in that** at least one from among said first tubular element (2) and said second tubular element (3) is formed in a single piece.

9. Device according to any previous claim, **characterized in that** said engagement means comprise at least one tab or protuberance (8) extending from said shank component (3d, 2d1) and a cap component (9) intended to engage said at least one tab or protuberance (8), so as to rotatably constrain said shank component (3d) to said cradle or annular component (2d) about said articulation axis (z-z).

10. Device for supplying fluid substances in the body of a patient, comprising:
- a first tubular element (2) having an end of connection (2a) to a source of a fluid substance and a supply end (2b), said first tubular element (2) defining a first flow channel (4) for conveying a fluid substance from said end of connection (2a) to said supply end (2b), said first tubular element (2) having at least one first segment (2c) extending along a first longitudinal axis (x-x);
- a second tubular element (3) having an inlet end (3a) as well as an outlet end (3b) for said fluid substance, said second tubular element (3) defining a second flow channel (5) in fluid communication with said first flow channel (4) and intended to convey a fluid substance from said inlet end (3a) to said outlet end (3b), said second tubular element (3) having at least one first section (3c) extending along a second longitudinal axis (y-y);
- at least one articulation or angular movement component (2d, 3d, 2d1, 3d1) for articulating or angularly moving said first tubular element (2) with respect to said second tubular element (3) around an articulation axis (z-z), said articulation axis being transverse or orthogonal to said first segment (2c) of said first tubular element (2), said at least one articulation or angular movement component (2d, 3d, 2d1, 3d1) extending along said articulation axis (z-z) so as to offset said first segment (2c) of said first tubular element (2) and said first section (3c) of said second tubular element (3) in the direction of said articulation axis (z-z), said at least one articulation or angular movement component (2d, 3d, 2d1, 3d1) comprising:
- a shank component (3d, 2d1) of one from among said first tubular element (2) and said second tubular element (3), said shank component (3d, 2d1) defining said inlet end (3a) or said supply end (2b), as well as
- a cradle or annular component (2d, 3d1) of the other from among said second tubular element (3) and said first tubular element (2), said cradle or annular component (2d, 3d1) defining the other from among said supply end (2b) or said inlet end (3a),
said cradle or annular component (2d, 3d1) delimiting a seat (6) for housing and articulated connection of said shank component (3d, 2d1) around said articulation axis (z-z), thereby allowing the articulation of said supply end (2b) with respect to said inlet end (3a) around said articulation axis (z-z),
wherein said first tubular element (2) or said second tubular element (3) is substantially configured as an elbow, one arm of said elbow comprising said first section (3c) or said first segment (2c), whereas the other arm of said elbow includes said shank component (3d, 2d1), and
wherein said device comprises engagement means (8, 9) for connecting, with rotatable engagement, said shank component (3d, 2d1) with said cradle or annular component (2d, 3d1), **characterized in that** said engagement means comprise at least one tab or protuberance (8) extending from said shank component (3d, 2d1) and a cap component (9) intended to engage said at least one tab or protuberance (8), so as to rotatably constrain said shank component (3d) to said cradle or annular component (2d) about said articulation axis (z-z).

11. Device according to claim 9 or 10, **characterized in that** said cap component (9) has a base segment (9a) as well as a stem segment (9b) insertable within said seat for housing and articulated connection (6), but on an opposite side with respect to the end (6a) of insertion in said seat (6) of said shank component (3d), so as to engage said shank component (3d) and maintain it inserted in position within said seat for housing and articulated connection (6).

12. Device according to any one of the preceding claims, comprising sealing means (13a, 13b) for the fluid seal connection between said first flow channel (4) and said second flow channel (5).

13. Device according to claims 1 and 12, **characterized in that** said sealing means comprise at least two annular gaskets (13a, 13b) mounted on said shank component (3d) so as to delimit with said cradle or annular component (2d) and with said shank component (3d) a first zone of said seat for housing and articulated connection (6), **in that** said first flow channel (4) opens in said first zone of said seat for housing and articulated connection (6) and **in that** said at least one through hole (5c) opens in said first zone.

14. Device according to claim 13, **characterized in that** it comprises at least four annular ribs (3e, 3f, 3g, 3h) suitably spaced from one another and each extending from the external wall of said shank component (3d) or from the internal surface of said cradle or annular component (2d), and **in that** each gasket (13a, 13b) is located between two respective annular ribs (3e, 3f, 3g, 3h) of said at least four annular ribs.

15. Device according to any one of the preceding claims, **characterized in that** it comprises a pressure gauge component (17) intended to measure the pressure of the fluid in said first (4) and/or in said second (5) flow channel.

16. Device according to claim 15, **characterized in that** said pressure gauge component comprises a manometer (17) and **in that** said device includes a support component (18) for said manometer (17) extending from said cradle or annular component (2d).

17. Device according to claim 16, **characterized in that** it comprises a membrane (19) housed between said support component (18) and said first (4) or second (5) flow channel, said membrane (19) being arranged to be stressed by the fluid flowing in said first (4) or second (5) flow channel, thereby determining a corresponding pressure measurement by said manometer (17).

18. Device according to any one of the preceding claims, **characterized in that** said first flow channel (4) has substantially constant diameter or section for the entire extension thereof and is substantially straight or rectilinear.

19. Device according to any one of the preceding claims, **characterized in that** said shank component (2d1) is part of said first tubular element (2) and is extended from said first segment (2c) of the latter, while said annular or cradle component (3d1) is part of said second tubular element (3) and is extended from said first section (3c) of the same.

20. Unit for supplying a fluid substance comprising at least one device according to any one of the preceding claims, at least one syringe (11) connected to said end of connection (2a) as well as a needle (12) connected to said outlet end (3b).

21. Unit for supplying according to claim 20, wherein said outlet end (3b) is removably fixed to said needle (21), in such a way that said second tubular component (3) and said needle (12) cannot rotate with respect to each other.

## Patentansprüche

1. Vorrichtung zum Zuführen von fluiden Substanzen in den Körper eines Patienten, umfassend:
- ein erstes rohrförmiges Element (2) mit einem Verbindungsende (2a) mit einer Quelle einer fluiden Substanz und einem Zufuhrende (2b), wobei das besagte erste rohrförmige Element (2) einen ersten Strömungskanal (4) zum Fördern einer fluiden Substanz von dem Verbindungsende (2a) zu dem besagten Zufuhrende (2b) definiert, wobei das besagte erste rohrförmige Element (2) mindestens ein erstes Segment (2c) aufweist, das sich entlang einer ersten Längsachse (x-x) erstreckt;
- ein zweites rohrförmiges Element (3) mit einem Einlassende (3a) sowie einem Auslassende (3b) für die besagte fluide Substanz, wobei das besagte zweite rohrförmige Element (3) einen zweiten Strömungskanal (5) in Fluidverbindung mit dem besagten ersten Strömungskanal (4) definiert und dazu bestimmt ist, eine fluide Substanz von dem besagten Einlassende (3a) zu dem besagten Auslassende (3b) zu fördern, wobei das besagte zweite rohrförmige Element (3) mindestens einen ersten Abschnitt (3c) aufweist, der sich entlang einer zweiten Längsachse (y-y) erstreckt;
- mindestens eine Gelenk- oder Winkelbewegungskomponente (2d, 3d, 2d1, 3d1) zum gelenkigen Bewegen oder Winkelbewegen des besagten ersten rohrförmigen Elements (2) in Bezug auf das besagte zweite rohrförmige Element (3) um eine Gelenkachse (z-z), wobei die besagte Gelenkachse quer oder orthogonal zu dem besagten ersten Segment (2c) des besagten ersten rohrförmigen Elements (2) verläuft, wobei sich die besagte mindestens eine Gelenk- oder Winkelbewegungskomponente (2d, 3d, 2d1, 3d1) entlang der besagten Gelenkachse (z-z) erstreckt, um das besagte erste Segment (2c) des besagten ersten rohrförmigen Elements (2) und den besagten ersten Abschnitt (3c) des besagten zweiten rohrförmigen Elements (3) in Richtung der besagten Gelenkachse (z-z) zu verschieben, wobei die besagte mindestens eine Gelenk- oder Winkelbewegungskomponente (2d, 3d, 2d1, 3d1) umfasst:
- eine Schaftkomponente (3d, 2d1) von einem aus dem besagten ersten rohrförmigen Element (2) und dem besagten zweiten rohrförmigen Element (3), wobei die besagte Schaftkomponente (3d, 2d1) das besagte Einlassende (3a) oder das besagte Zufuhrende (2b) definiert, sowie
- eine Halterung oder ringförmige Komponente (2d, 3d1) des anderen aus dem besagten zweiten rohrförmigen Element (3) und dem besagten ersten rohrförmigen Element (2), wobei die besagte Halterung oder ringförmige Komponente (2d, 3d1) das andere aus dem besagten Zufuhrende (2b) oder dem besagten Einlassende (3a) definiert,
die besagte Halterung oder ringförmige Komponente (2d, 3d1) begrenzend einen Sitz (6) zur Aufnahme und gelenkigen Verbindung der besagten Schaftkomponente (3d, 2d1) um die besagte Gelenkachse (z-z), wodurch die Gelenkbewegung des besagten Zufuhrendes (2b) in Bezug auf das besagte Einlassende (3a) um die Gelenkachse (z-z) ermöglicht wird,
worin das erste rohrförmige Element (2) oder das besagte zweite rohrförmige Element (3) im Wesentlichen als ein Ellenbogen konfiguriert ist, wobei ein Arm des besagten Ellenbogens den besagten ersten Abschnitt (3c) oder das besagte erste Segment (2c) umfasst, wohingegen der andere Arm des besagten Ellenbogens die besagte Schaftkomponente (3d, 2d1) enthält,
worin die besagte Vorrichtung Eingriffmittel (8, 9) zum Verbinden, mit drehbarem Eingriff, der besagten Schaftkomponente (3d, 2d1) mit der besagten Halterung oder ringförmigen Komponente (2d, 3d1) umfasst,
worin das besagte zweite rohrförmige Element (3), vom besagten Auslassende (3b) zum besagten Einlassende (3a), den besagten ersten Abschnitt (3c) und die besagte Schaftkomponente (3d) umfasst, wobei sich die besagte Schaftkomponente (3d) quer oder orthogonal vom ersten Abschnitt (3c) erstreckt und sowohl einen jeweiligen ersten (5a) als auch zweiten (5b) Zweig, quer oder orthogonal zueinander, des besagten zweiten Strömungskanals (5) begrenzt, und
worin die besagte Schaftkomponente (3d) eine Innenwand, die den besagten zweiten Zweig (5b) des besagten zweiten Strömungskanals (5) begrenzt, und eine Außenwand hat, die so geformt ist, dass sie in den besagten Sitz zur Aufnahme und gelenkigen Verbindung (6) einsetzbar ist, **dadurch gekennzeichnet, dass** in der Wand der besagten Schaftkomponente (3d) mindestens ein Durchgangsloch (5c) ausgebildet ist, das dazu bestimmt ist, die Zone außerhalb der besagten Schaftkomponente (3d) und den besagten zweiten Strömungskanal (5) in Fluidverbindung zu bringen.

2. Vorrichtung nach Anspruch 1, **dadurch gekennzeichnet, dass** die besagte mindestens eine Gelenk- oder Winkelbewegungskomponente (2d, 3d, 2d1, 3d1) quer oder orthogonal zu dem besagten mindestens einen ersten Segment (2c) des besagten ersten rohrförmigen Elements (2) und zu dem besagten ersten Abschnitt (3c) des besagten zweiten rohrförmigen Elements (3) ist.

3. Vorrichtung nach Anspruch 1 oder 2, **dadurch gekennzeichnet, dass** das besagte erste Segment (2c) des besagten ersten rohrförmigen Elements (2) in Bezug auf das zweite besagte rohrförmige Element (3) derart gelenkig verbunden ist, dass eine Gelenkebene (PA) definiert wird, die orthogonal zu der besagten Gelenkachse (z-z) ist und durch die besagte erste Längsachse (x-x) verläuft, und dadurch, dass der besagte erste Abschnitt (3c) des besagten zweiten rohrförmigen Elements (3) in Bezug auf die besagte Gelenkebene (PA) verschoben ist.

4. Vorrichtung nach irgendeinem der vorangegangenen Ansprüche, **dadurch gekennzeichnet, dass** das besagte Einlassende (3a) des besagten zweiten rohrförmigen Elements (3) mit dem besagte Zufuhrende (2b) des besagten ersten rohrförmigen Elements (2) um die besagte Gelenkachse (z-z) gelenkig verbunden ist.

5. Vorrichtung nach irgendeinem der vorangegangenen Ansprüche, **dadurch gekennzeichnet, dass** sie Mittel zum lösbaren Befestigen des besagten Auslassendes (3b) an einer Nadel (12) umfasst.

6. Vorrichtung nach irgendeinem der vorangegangenen Ansprüche, **dadurch gekennzeichnet, dass**, von dem besagten Verbindungsende (2a) mit dem besagten Zufuhrende (2b), das besagte erste rohrförmige Element (2) das besagte erste Segment (2c) umfasst und dann die besagte Halterung oder ringförmige Komponente (2d), wobei die besagte Halterung oder ringförmige Komponente (2d) ein im Wesentlichen zylindrisches Segment enthält, das sich quer oder orthogonal ausgehend vom Abschlussende des besagten ersten Segments (2c) erstreckt und innen den besagten Sitz zur Aufnahme und gelenkigen Verbindung (6) definiert.

7. Vorrichtung nach Anspruch 6, **dadurch gekennzeichnet, dass** das besagte im Wesentlichen zylindrische Segment und der besagte Sitz (6) zur Aufnahme und gelenkigen Verbindung eine Längssymmetrieachse haben, die mit der besagten Gelenkachse (z-z) übereinstimmt.

8. Vorrichtung nach irgendeinem der vorangegangenen Ansprüche, **dadurch gekennzeichnet, dass** mindestens eines aus dem besagten ersten rohrförmigen Element (2) und dem besagten zweiten rohrförmigen Element (3) einstückig ausgebildet ist.

9. Vorrichtung nach irgendeinem vorangegangenen Anspruch, **dadurch gekennzeichnet, dass** die besagten Eingriffsmittel mindestens einen Ansatz oder Vorsprung (8), der sich von der besagten Schaftkomponente (3d, 2d1) erstreckt, und eine Kappenkomponente (9), die dazu bestimmt ist, in den besagten mindestens eine Ansatz oder Vorsprung (8) einzugreifen, umfassen, um die besagte Schaftkomponente (3d) an der besagten Halterung oder ringförmigen Komponente (2d) um die Gelenkachse (z-z) drehbar festzuhalten.

10. Vorrichtung zum Zuführen von fluiden Substanzen in den Körper eines Patienten, umfassend:
- ein erstes rohrförmiges Element (2) mit einem Verbindungsende (2a) mit einer Quelle einer fluiden Substanz und einem Zufuhrende (2b), wobei das besagte erste rohrförmige Element (2) einen ersten Strömungskanal (4) zum Fördern einer fluiden Substanz von dem Verbindungsende (2a) zu dem besagten Zufuhrende (2b) definiert, wobei das besagte erste rohrförmige Element (2) mindestens ein erstes Segment (2c) aufweist, das sich entlang einer ersten Längsachse (x-x) erstreckt;
- ein zweites rohrförmiges Element (3) mit einem Einlassende (3a) sowie einem Auslassende (3b) für die besagte fluide Substanz, wobei das besagte zweite rohrförmige Element (3) einen zweiten Strömungskanal (5) in Fluidverbindung mit dem besagten ersten Strömungskanal (4) definiert und dazu bestimmt ist, eine fluide Substanz von dem besagten Einlassende (3a) zu dem besagten Auslassende (3b) zu fördern, wobei das besagte zweite rohrförmige Element (3) mindestens einen ersten Abschnitt (3c) aufweist, der sich entlang einer zweiten Längsachse (y-y) erstreckt;
- mindestens eine Gelenk- oder Winkelbewegungskomponente (2d, 3d, 2d1, 3d1) zum gelenkigen Bewegen oder Winkelbewegen des besagten ersten rohrförmigen Elements (2) in Bezug auf das besagte zweite rohrförmige Element (3) um eine Gelenkachse (z-z), wobei die besagte Gelenkachse quer oder orthogonal zu dem besagten ersten Segment (2c) des besagten ersten rohrförmigen Elements (2) verläuft, wobei sich die besagte mindestens eine Gelenk- oder Winkelbewegungskomponente (2d, 3d, 2d1, 3d1) entlang der besagten Gelenkachse (z-z) erstreckt, um das besagte erste Segment (2c) des besagten ersten rohrförmigen Elements (2) und den besagten ersten Abschnitt (3c) des besagten zweiten rohrförmigen Elements (3) in Richtung der besagten Gelenkachse (z-z) zu verschieben, wobei die besagte mindestens eine Gelenk- oder Winkelbewegungskomponente (2d, 3d, 2d1, 3d1) umfasst:
- eine Schaftkomponente (3d, 2d1) von einem aus dem besagten ersten rohrförmigen Element (2) und dem besagten zweiten rohrförmigen Element (3), wobei die besagte Schaftkomponente (3d, 2d1) das besagte Einlassende (3a) oder das besagte Zufuhrende (2b) definiert, sowie
- eine Halterung oder ringförmige Komponente (2d, 3d1) des anderen aus dem besagten zweiten rohrförmigen Element (3) und dem besagten ersten rohrförmigen Element (2), wobei die besagte Halterung oder ringförmige Komponente (2d, 3d1) das andere aus dem besagten Zufuhrende (2b) oder dem besagten Einlassende (3a) definiert,
die besagte Halterung oder ringförmige Komponente (2d, 3d1) begrenzend einen Sitz (6) zur Aufnahme und gelenkigen Verbindung der besagten Schaftkomponente (3d, 2d1) um die besagte Gelenkachse (z-z), wodurch die Gelenkbewegung des besagten Zufuhrendes (2b) in Bezug auf das besagte Einlassende (3a) um die Gelenkachse (z-z) ermöglicht wird,
worin das besagte erste rohrförmige Element (2) oder das besagte zweite rohrförmige Element (3) im Wesentlichen als ein Ellenbogen konfiguriert ist, wobei ein Arm des besagten Ellenbogens den besagten ersten Abschnitt (3c) oder das besagte erste Segment (2c) umfasst, wohingegen der andere Arm des besagten Ellenbogens die besagte Schaftkomponente (3d, 2d1) enthält, und
worin die besagte Vorrichtung Eingriffmittel (8, 9) zum Verbinden, mit drehbarem Eingriff, der besagten Schaftkomponente (3d, 2d1) mit der besagten Halterung oder ringförmigen Komponente (2d, 3d1) umfasst, **dadurch gekennzeichnet, dass**
die besagten Eingriffsmittel mindestens einen Ansatz oder Vorsprung (8), der sich von der besagten Schaftkomponente (3d, 2d1) erstreckt, und eine Kappenkomponente (9), die dazu bestimmt ist, in den besagten mindestens eine Ansatz oder Vorsprung (8) einzugreifen, umfassen, um die besagte Schaftkomponente (3d) an der besagten Halterung oder ringförmigen Komponente (2d) um die Gelenkachse (z-z) drehbar festzuhalten.

11. Vorrichtung nach Anspruch 9 oder 10, **dadurch gekennzeichnet, dass** die besagte Kappenkomponente (9) ein Basissegment (9a) sowie ein Stangensegment (9b) hat, das in den besagten Sitz zur Aufnahme und gelenkigen Verbindung (6) einsetzbar ist, allerdings auf einer gegenüberliegenden Seite in Bezug auf das Ende (6a) des Einsetzens in den besagten Sitz (6) der besagten Schaftkomponente (3d), um die besagte Schaftkomponente (3d) in Eingriff zu nehmen und sie in dem besagten Sitz zur Aufnahme und gelenkigen Verbindung (6) in Position eingesetzt zu halten.

12. Vorrichtung nach irgendeinem der vorangegangenen Ansprüche, umfassend Dichtungsmittel (13a, 13b) für die fluiddichte Verbindung zwischen dem besagten ersten Strömungskanal (4) und dem besagten zweiten Strömungskanal (5).

13. Vorrichtung nach den Ansprüchen 1 und 12, **dadurch gekennzeichnet, dass** die besagten Dichtungsmittel mindestens zwei ringförmige Dichtungen (13a, 13b) umfassen, die auf der besagten Schaftkomponente (3d) montiert sind, um mit der besagten Halterung oder ringförmigen Komponente (2d) und mit der besagten Schaftkomponente (3d) eine erste Zone des besagten Sitzes zur Aufnahme und gelenkigen Verbindung (6) zu begrenzen, dadurch, dass sich der besagte erste Strömungskanal (4) in der ersten Zone des besagten Sitzes zur Aufnahme und gelenkigen Verbindung (6) öffnet und dadurch, dass sich das besagte mindestens eine Durchgangsloch (5c) in der besagten ersten Zone öffnet.

14. Vorrichtung nach Anspruch 13, **dadurch gekennzeichnet, dass** sie mindestens vier ringförmige Rippen (3e, 3f, 3g, 3h) umfasst, die in angemessenem Abstand voneinander angeordnet sind und sich jeweils von der Außenwand der besagten Schaftkomponente (3d) oder von der Innenfläche der besagten Halterung oder der ringförmigen Komponente (2d) erstrecken, und dadurch, dass jede Dichtung (13a, 13b) zwischen zwei jeweiligen ringförmigen Rippen (3e, 3f, 3g, 3h) der besagten mindestens vier ringförmigen Rippen angeordnet ist.

15. Vorrichtung nach irgendeinem der vorangegangenen Ansprüche, **dadurch gekennzeichnet, dass** sie eine Druckmesser-Komponente (17) umfasst, die dazu bestimmt ist, den Druck des Fluids in dem besagten ersten (4) und/oder in dem besagten zweiten (5) Strömungskanal zu messen.

16. Vorrichtung nach Anspruch 15, **dadurch gekennzeichnet, dass** die besagte Druckmesser-Komponente ein Manometer (17) umfasst und dadurch, dass die besagte Vorrichtung eine Stützkomponente (18) für das besagte Manometer (17) enthält, die sich von der besagten Halterung oder ringförmigen Komponente (2d) erstreckt.

17. Vorrichtung nach Anspruch 16, **dadurch gekennzeichnet, dass** sie eine Membran (19) umfasst, die zwischen der besagten Stützkomponente (18) und dem besagten ersten (4) oder zweiten (5) Strömungskanal untergebracht ist, wobei die besagte Membran (19) so angeordnet ist, dass sie durch das in dem besagten ersten (4) oder zweiten (5) Strömungskanal strömende Fluid beansprucht wird, wodurch eine entsprechende Druckmessung durch das besagte Manometer (17) verursacht wird.

18. Vorrichtung nach irgendeinem der vorangegangenen Ansprüche, **dadurch gekennzeichnet, dass** der besagte erste Strömungskanal (4) einen im Wesentlichen konstanten Durchmesser oder Querschnitt über die gesamte Erstreckung hinweg aufweist und im Wesentlichen gerade oder geradlinig ist.

19. Vorrichtung nach irgendeinem der vorangegangenen Ansprüche, **dadurch gekennzeichnet, dass** die besagte Schaftkomponente (2d1) Teil des besagten ersten rohrförmigen Elements (2) ist und sich von dem besagten ersten Segment (2c) des letzteren erstreckt, während die besagte ringförmige oder Halterungskomponente (3d1) Teil des besagten zweiten rohrförmigen Elements (3) ist und sich von dem besagten ersten Abschnitt (3c) desselben erstreckt.

20. Einheit zum Zuführen einer fluiden Substanz, umfassend mindestens eine Vorrichtung nach irgendeinem der vorangegangenen Ansprüche, mindestens eine Spritze (11), die mit dem besagten Verbindungsende (2a) verbunden ist, sowie eine Nadel (12), die mit dem besagten Auslassende (3b) verbunden ist.

21. Einheit zum Zuführen nach Anspruch 20, wobei das besagte Auslassende (3b) an der besagten Nadel (21) derart lösbar befestigt ist, dass sich die besagte zweite rohrförmige Komponente (3) und die besagte Nadel (12) in Bezug aufeinander nicht drehen können.

## Revendications

1. Dispositif pour alimenter des substances fluidiques dans le corps d'un patient, comprenant :
- un premier élément tubulaire (2) comportant une extrémité de raccordement (2a) à une source de substance fluidique et une extrémité d'alimentation (2b), ledit premier élément tubulaire (2) définissant un premier canal d'écoulement (4) pour transporter une substance fluidique de ladite extrémité de raccordement (2a) à ladite extrémité d'alimentation (2b), ledit premier élément tubulaire (2) comportant au moins un premier segment (2c) s'étendant le long d'un premier axe longitudinal (x-x) ;
- un deuxième élément tubulaire (3) comportant une extrémité d'entrée (3a) ainsi qu'une extrémité de sortie (3b) pour ladite substance fluidique, ledit deuxième élément tubulaire (3) définissant un deuxième canal d'écoulement (5) en communication fluidique avec ledit premier canal d'écoulement (4) et destiné à transporter une substance fluidique de ladite extrémité d'entrée (3a) à ladite extrémité de sortie (3b), ledit deuxième élément tubulaire (3) comportant au moins une première section (3c) s'étendant le long d'un deuxième axe longitudinal (y-y) ;
- au moins un composant d'articulation ou de déplacement angulaire (2d, 3d, 2d1, 3d1) pour articuler ou déplacer angulairement ledit premier élément tubulaire (2) par rapport audit deuxième élément tubulaire (3) autour d'un axe d'articulation (z-z), ledit axe d'articulation étant transversal ou orthogonal audit premier segment (2c) dudit premier élément tubulaire (2), ledit au moins un composant d'articulation ou de déplacement angulaire (2d, 3d, 2d1, 3d1) s'étendant le long dudit axe d'articulation (z-z) de façon à compenser ledit premier segment (2c) dudit premier élément tubulaire (2) et ladite première section (3c) dudit deuxième élément tubulaire (3) dans la direction dudit axe d'articulation (z-z), ledit au moins un composant d'articulation ou de déplacement angulaire (2d, 3d, 2d1, 3d1) comprenant :
- un composant de tige (3d, 2d1) de l'un parmi ledit premier élément tubulaire (2) et ledit deuxième élément tubulaire (3), ledit composant de tige (3d, 2d1) définissant ladite extrémité d'entrée (3a) ou ladite extrémité d'alimentation (2b), ainsi que
- un composant en berceau ou annulaire (2d, 3d1) de l'autre parmi ledit deuxième élément tubulaire (3) et ledit premier élément tubulaire (2), ledit composant en berceau ou annulaire (2d, 3d1) définissant l'autre parmi ladite extrémité d'alimentation (2b) ou ladite extrémité d'entrée (3a),
ledit composant en berceau ou annulaire (2d, 3d1) délimitant un siège (6) de logement et raccordement articulé dudit composant de tige (3d, 2d1) autour dudit axe d'articulation (z-z), permettant ainsi l'articulation de ladite extrémité d'alimentation (2b) par rapport à ladite extrémité d'entrée (3a) autour dudit axe d'articulation (z-z),
dans lequel ledit premier élément tubulaire (2) ou ledit deuxième élément tubulaire (3) est sensiblement configuré en forme de coude, un bras dudit coude comprenant ladite première section (3c) ou ledit premier segment (2c), alors que l'autre bras dudit coude comprend ledit composant de tige (3d, 2d1),
dans lequel ledit dispositif comprend des moyens d'accouplement (8, 9) pour raccorder, par un accouplement rotatif, ledit composant de tige (3d, 2d1) audit composant en berceau ou annulaire (2d, 3d1),
dans lequel ledit deuxième élément tubulaire (3) comprend, de ladite extrémité de sortie (3b) à ladite extrémité d'entrée (3a), ladite première section (3c) et ledit composant de tige (3d), ledit composant de tige (3d) s'étendant transversalement ou orthogonalement depuis la première section (3c) et délimitant tous les deux une première (5a) et une deuxième (5b) dérivation respectives, transversales ou orthogonales l'une à l'autre, dudit deuxième canal d'écoulement (5), et
dans lequel ledit composant de tige (3d) comporte une paroi intérieure délimitant ladite deuxième dérivation (5b) dudit deuxième canal d'écoulement (5) et une paroi extérieure formée pour pouvoir être insérée dans ledit siège de logement et raccordement articulé (6), **caractérisé en ce que** dans la paroi dudit composant de tige (3d) au moins un trou traversant (5c) est formé, destiné à mettre en communication fluidique la zone à l'extérieur dudit composant de tige (3d) et ledit deuxième canal d'écoulement (5).

2. Dispositif selon la revendication 1, **caractérisé en ce que** ledit au moins un composant d'articulation ou de mouvement angulaire (2d, 3d, 2d1, 3d1) est transversal ou orthogonal audit au moins un premier segment (2c) dudit premier élément tubulaire (2) et à ladite première section (3c) dudit deuxième élément tubulaire (3) .

3. Dispositif selon la revendication 1 ou 2, **caractérisé en ce que** ledit premier segment (2c) dudit premier élément tubulaire (2) est articulé par rapport audit deuxième élément tubulaire (3) de manière à définir un plan d'articulation (PA) orthogonal audit axe d'articulation (z-z) et traversant ledit premier axe longitudinal (x-x), et **en ce que** ladite première section (3c) dudit deuxième élément tubulaire (3) est décalée par rapport audit plan d'articulation (PA).

4. Dispositif selon l'une quelconque des revendications précédentes, **caractérisé en ce que** ladite extrémité d'entrée (3a) dudit deuxième élément tubulaire (3) est articulée sur ladite extrémité d'alimentation (2b) dudit premier élément tubulaire (2) autour dudit axe d'articulation (z-z).

5. Dispositif selon l'une quelconque des revendications précédentes, **caractérisé en ce qu'**il comprend des moyens pour fixer de manière amovible ladite extrémité de sortie (3b) à une aiguille (12).

6. Dispositif selon l'une quelconque des revendications précédentes, **caractérisé en ce que** de ladite extrémité de raccordement (2a) à ladite extrémité d'alimentation (2b), ledit premier élément tubulaire (2) comprend ledit premier segment (2c) et puis ledit composant en berceau ou annulaire (2d), ledit composant en berceau ou annulaire (2d) comprenant un segment sensiblement cylindrique étendu transversalement ou orthogonalement à partir de l'extrémité terminale dudit premier segment (2c) et définissant à l'intérieur ledit siège de logement et raccordement articulé (6).

7. Dispositif selon la revendication 6, **caractérisé en ce que** ledit segment sensiblement cylindrique et ledit siège (6) de logement et raccordement articulé ont un axe de symétrie longitudinal coïncidant avec ledit axe d'articulation (z-z).

8. Dispositif selon l'une quelconque des revendications précédentes, **caractérisé en ce qu'**au moins l'un parmi ledit premier élément tubulaire (2) et ledit deuxième élément tubulaire (3) est formé d'une seule partie.

9. Dispositif selon l'une quelconque des revendications précédentes, **caractérisé en ce que** lesdits moyens d'accouplement comprennent au moins un tenon ou une protubérance (8) s'étendant depuis ledit composant de tige (3d, 2d1) et un composant de capuchon (9) destiné à s'accoupler avec lesdits au moins un tenon ou une protubérance (8), de façon à maintenir de manière rotative ledit composant de tige (3d) sur ledit composant en berceau ou annulaire (2d) autour dudit axe d'articulation (z-z).

10. Dispositif pour alimenter des substances fluidiques dans le corps d'un patient, comprenant :
- un premier élément tubulaire (2) comportant une extrémité de raccordement (2a) à une source de substance fluidique et une extrémité d'alimentation (2b), ledit premier élément tubulaire (2) définissant un premier canal d'écoulement (4) pour transporter une substance fluidique de ladite extrémité de raccordement (2a) à ladite extrémité d'alimentation (2b), ledit premier élément tubulaire (2) comportant au moins un premier segment (2c) s'étendant le long d'un premier axe longitudinal (x-x) ;
- un deuxième élément tubulaire (3) comportant une extrémité d'entrée (3a) ainsi qu'une extrémité de sortie (3b) pour ladite substance fluidique, ledit deuxième élément tubulaire (3) définissant un deuxième canal d'écoulement (5) en communication fluidique avec ledit premier canal d'écoulement (4) et destiné à transporter une substance fluidique de ladite extrémité d'entrée (3a) à ladite extrémité de sortie (3b), ledit deuxième élément tubulaire (3) comportant au moins une première section (3c) s'étendant le long d'un deuxième axe longitudinal (y-y) ;
- au moins un composant d'articulation ou de déplacement angulaire (2d, 3d, 2d1, 3d1) pour articuler ou déplacer angulairement ledit premier élément tubulaire (2) par rapport audit deuxième élément tubulaire (3) autour d'un axe d'articulation (z-z), ledit axe d'articulation étant transversal ou orthogonal audit premier segment (2c) dudit premier élément tubulaire (2), ledit au moins un composant d'articulation ou de déplacement angulaire (2d, 3d, 2d1, 3d1) s'étendant le long dudit axe d'articulation (z-z) de façon à compenser ledit premier segment (2c) dudit premier élément tubulaire (2) et ladite première section (3c) dudit deuxième élément tubulaire (3) dans la direction dudit axe d'articulation (z-z), ledit au moins un composant d'articulation ou de déplacement angulaire (2d, 3d, 2d1, 3d1) comprenant :
- un composant de tige (3d, 2d1) de l'un parmi ledit premier élément tubulaire (2) et ledit deuxième élément tubulaire (3), ledit composant de tige (3d, 2d1) définissant ladite extrémité d'entrée (3a) ou ladite extrémité d'alimentation (2b), ainsi que
- un composant en berceau ou annulaire (2d, 3d1) de l'autre parmi ledit deuxième élément tubulaire (3) et ledit premier élément tubulaire (2), ledit composant en berceau ou annulaire (2d, 3d1) définissant l'autre parmi ladite extrémité d'alimentation (2b) ou ladite extrémité d'entrée (3a),
ledit composant en berceau ou annulaire (2d, 3d1) délimitant un siège (6) de logement et raccordement articulé dudit composant de tige (3d, 2d1) autour dudit axe d'articulation (z-z), permettant ainsi l'articulation de ladite extrémité d'alimentation (2b) par rapport à ladite extrémité d'entrée (3a) autour dudit axe d'articulation (z-z),
dans lequel ledit premier élément tubulaire (2) ou ledit deuxième élément tubulaire (3) est sensiblement configuré en forme de coude, un bras dudit coude comprenant ladite première section (3c) ou ledit premier segment (2c), alors que l'autre bras dudit coude comprend ledit composant de tige (3d, 2d1) et dans lequel ledit dispositif comprend des moyens d'accouplement (8, 9) pour raccorder, par un accouplement rotatif, ledit composant de tige (3d, 2d1) audit composant en berceau ou annulaire (2d, 3d1), **caractérisé en ce que**
lesdits moyens d'accouplement comprennent au moins un tenon ou une protubérance (8) s'étendant depuis ledit composant de tige (3d, 2d1) et un composant de capuchon (9) destiné à s'accoupler avec lesdits au moins un tenon ou une protubérance (8), de façon à maintenir de manière rotative ledit composant de tige (3d) sur ledit composant en berceau ou annulaire (2d) autour dudit axe d'articulation (z-z).

11. Dispositif selon la revendication 9 ou 10, **caractérisé en ce que** ledit composant de capuchon (9) comporte un segment de base (9a) ainsi qu'un segment de pied (9b) pouvant être inséré dans ledit siège de logement et raccordement articulé (6), mais sur un côté opposé par rapport à l'extrémité (6a) d'insertion dans ledit siège (6) dudit composant de tige (3d), de façon à accoupler ledit composant de tige (3d) et le maintenir inséré en position dans ledit siège de logement et raccordement articulé (6).

12. Dispositif selon l'une quelconque des revendications précédentes, comprenant des moyens d'étanchéité (13a, 13b) pour le raccordement étanche fluidique entre ledit premier canal d'écoulement (4) et ledit deuxième canal d'écoulement (5).

13. Dispositif selon les revendications 1 et 12, **caractérisé en ce que** lesdits moyens d'étanchéité comprennent au moins deux joints annulaires (13a, 13b) montés sur ledit composant de tige (3d) de façon à délimiter avec ledit composant en berceau ou annulaire (2d) et avec ledit composant de tige (3d) une première zone dudit siège de logement et raccordement articulé (6), **en ce que** ledit premier canal d'écoulement (4) s'ouvre dans ladite première zone dudit siège de logement et raccordement articulé (6) et **en ce que** ledit au moins un trou traversant (5c) s'ouvre dans ladite première zone.

14. Dispositif selon la revendication 13, **caractérisé en ce qu'**il comprend au moins quatre nervures annulaires (3e, 3f, 3g, 3h) convenablement espacées l'une de l'autre et s'étendant chacune à partir de la paroi extérieure dudit composant de tige (3d) ou de la surface interne dudit composant en berceau ou annulaire (2d), et **en ce que** chaque joint (13a, 13b) est situé entre deux nervures annulaires (3e, 3f, 3g, 3h) respectives desdites au moins quatre nervures annulaires.

15. Dispositif selon l'une quelconque des revendications précédentes, **caractérisé en ce qu'**il comprend un composant indicateur de pression (17) destiné à mesurer la pression du fluide dans ledit premier (4) et/ou dans ledit deuxième (5) canal d'écoulement.

16. Dispositif selon la revendication 15, **caractérisé en ce que** ledit composant indicateur de pression comprend un manomètre (17) et **en ce que** ledit dispositif comprend un composant de support (18) pour ledit manomètre (17) s'étendant depuis ledit composant en berceau ou annulaire (2d).

17. Dispositif selon la revendication 16, **caractérisé en ce qu'**il comprend une membrane (19) contenue entre ledit composant de support (18) et ledit premier (4) ou deuxième (5) canal d'écoulement, ladite membrane (19) étant agencée pour être contrainte par le fluide s'écoulant dans ledit premier (4) ou deuxième (5) canal d'écoulement, déterminant ainsi une mesure de la pression correspondante avec ledit manomètre (17).

18. Dispositif selon l'une quelconque des revendications précédentes, **caractérisé en ce que** ledit premier canal d'écoulement (4) a un diamètre ou une section sensiblement constant/e sur toute son extension et est sensiblement droit ou rectiligne.

19. Dispositif selon l'une quelconque des revendications précédentes, **caractérisé en ce que** ledit composant de tige (2dl) fait partie dudit premier élément tubulaire (2) et s'étend depuis ledit premier segment (2c) de ce dernier, alors que ledit composant annulaire ou en berceau (3d1) fait partie dudit deuxième élément tubulaire (3) et s'étend depuis ladite première section (3c) de celui-ci.

20. Unité d'alimentation d'une substance fluidique comprenant au moins un dispositif selon l'une quelconque des revendications précédentes, au moins une seringue (11) raccordée à ladite extrémité de raccordement (2a) ainsi qu'une aiguille (12) raccordée à ladite extrémité de sortie (3b).

21. Unité d'alimentation selon la revendication 20, dans laquelle ladite extrémité de sortie (3b) est fixée de manière amovible à ladite aiguille (21), de telle manière que ledit deuxième composant tubulaire (3) et ladite aiguille (12) ne puissent pas tourner l'un par rapport à l'autre.
